# EUROPÄISCHE PATENTSCHRIFT

(11) **EP 3 622 000 B1**
(45) Veröffentlichungstag und Bekanntmachung des Hinweises auf die Patenterteilung: **28.04.2021**
(21) Anmeldenummer: 18719592.0
(22) Anmeldetag: 02.05.2018
(51) Int. Cl.: C08G 63/181, A61Q 5/06, A61K 8/85, C08G 63/688, C09J 167/02

(54) **EIN POLYESTER**
A POLYESTER
POLYESTER

(30) Priorität: 10.05.2017 EP 17170394
(43) Veröffentlichungstag der Anmeldung: 18.03.2020
(73) Patentinhaber: BASF SE, 67056 Ludwigshafen am Rhein (DE)
(72) Erfinder: SCHICK, Michael Bernhard, 67056 Ludwigshafen (DE); YAMAMOTO, Motonori, 67056 Ludwigshafen (DE)
(74) Vertreter: BASF IP Association
(86) Internationale Anmeldenummer: PCT/EP2018/061104
(87) Internationale Veröffentlichungsnummer: WO 2018/206349

(56) Entgegenhaltungen:
- WO-A1-2017/023175
- CN-A- 102 757 552

## Beschreibung

Die vorliegende Erfindung betrifft einen Polyester bestehend aus (A) Wiederholungseinheiten abgeleitet von einer Säurekomponente, welche besteht aus (a1) 2,5-Furandicarbonsäure, (a2) einer aliphatischen C₄ -C₃₆-Dicarbonsäure oder eine Mischung aus mehreren aliphatischen C₄ -C₃₆-Dicarbonsäuren, und (a3) einer sulfonatgruppenhaltigen Dicarbonsäure, sowie aus (B) Wiederholungseinheiten abgeleitet von einer Di-ol/amin-komponente, sowie optional aus weiteren Widerholungseinheiten (C) und/oder Verzweigern (E), sowie aus (D) Wiederholungseinheiten abgeleitet von mindestens einer di- oder oligofunktionellen Verbindung ausgewählt aus der Gruppe bestehend aus einem Di- oder Oligo-Isocyanat und einem Di- oder Oligo-Isocyanurat. Weiterhin betrifft die vorliegende Erfindung die Herstellung dieser Polyester und deren Verwendung.

Furandicarbonsäurehaltige aromatische Polyester sind beispielsweise aus der WO 2010/077133 bekannt und Furandicarbonsäurehaltige aliphatisch-aromatische Polyester aus der WO 2009/135921**.** Diese Polyester sind jedoch in Wasser nicht löslich Die WO 2017/023175 A1 offenbart einen Polyester, der durch Umsetzung eines Furandicarbonsäure-haltigen Polyesters mit einem Kettenverlängerer erhalten wird.

Aus CN 102757552 sind wasserlösliche, Furandicarbonsäurehaltige aromatische Polyester bekannt, die einen hohen Gehalt einer sulfonatgruppenhaltigen Verbindung von in den Beispielen über 12 mol-% aufweisen.

2,5-Furandicarbonsäure ist aus nachwachsenden Rohstoffen herstellbar. Polymere auf der Basis von 2,5-Furandicarbonsäure sind in vielen Fällen biologisch abbaubar. Für viele Anwendungen sind wasserlösliche Polymere erforderlich. Wasserlösliche Polymere auf der Basis von 2,5-Furandicarbonsäure sind aus CN 102757552 bekannt, sie enthalten neben 2,5-Furandicarbonsäure auch Natriumsulfoisophthalsäure. Allerdings sind die mechanischen Eigenschaften dieser Polymere für viele Anwendungen nicht ausreichend gut.

Der vorliegenden Erfindung liegt daher die Aufgabe zugrunde, wasserlösliche Polymere auf der Basis von 2,5-Furandicarbonsäure bereit zu stellen, die hohe mechanische Festigkeitswerte, wie z. B. eine hohe Streckspannung, aufweisen, so dass sich diese Polyester für Anwendungen eignen, die hohe mechanische Festigkeitswerte erfordern.

Diese Aufgabe wird gelöst durch den Polyester gemäß Anspruch 1, der ein Gegenstand der vorliegenden Erfindung ist. Die Unteransprüche stellen besondere Ausführungsformen der vorliegenden Erfindung dar. Die Nebenansprüche, gerichtet auf Produkte enthaltend den erfindungsgemäßen Polyester, Verwendungen enthaltend den erfindungsgemäßen Polyester und ein Verfahren zur Herstellung des erfindungsgemäßen Polyesters betreffen weitere Gegenstände der vorliegenden Erfindung.

Die erfindungsgemäßen Polyester sind in der Regel wasserlöslich. Im Vergleich zu den aus WO 2010/077133 und aus WO 2009/135921 bekannten Polyester weisen die erfindungsgemäßen Polyester in der Regel eine höhere biologische Abbaubarkeit und verbesserte mechanische Eigenschaften wie eine erhöhte Zugfestigkeit auf.

Im Folgenden wird die Erfindung näher beschrieben. Im Zweifel beziehen sich die folgenden Ausführungen nur auf besondere Ausführungsformen der vorliegenden Erfindung und schränken die Gegenstände der vorliegenden Erfindung nicht ein.

Die erfindungsgemäßen Polyester umfassen als besondere Ausführungsform aromatische Polyester enthaltend 100 mol-%, bezogen auf die Komponenten a1) und a2), 2,5-Furandicarbonsäure und damit keine Komponente a2) sowie aliphatisch-aromatische Polyester enthaltend 25 bis 99 mol-%, vorzugsweise 60 bis 75 ml-% und insbesondere bevorzugt 65 bis 75 mol-%, jeweils bezogen auf die Komponenten a1) und a2), 2,5-Furandicarbonsäure und entsprechend 1 bis 75 mol-%, vorzugsweise 25 bis 40 mol-% und insbesondere bevorzugt 25 bis 35 mol-% einer aliphatischen C₄-C₃₆-Dicarbonsäure, jeweils bezogen auf die Komponenten a1) und a2).

2,5-Furandicarbonsäure (Komponente a1) ist beispielsweise aus WO 2009/135921 bekannt.

Die 2,5-Furandicarbonsäure wird in einer Ausführungsform in die Polyestersynthese nicht als freie Säure, sondern als Di-C₁-C₈-Alkyester eingesetzt, wobei der 2,5-Furandicarbonsäurediethylester und insbesondere der 2,5-Furandicarbonsäure-dimethylester besonders bevorzugt sind.

In den aliphatisch-aromatischen Polyestern kommen als aliphatische C₄-C₃₆-Dicarbonsäuren (Komponente a2) insbesondere α,ω- C₄-C₃₆-Dicarbonsäuren in Frage und hier beispielsweise Bernsteinsäure, Glutarsäure, Adipinsäure, Korksäure, Azelainsäure, Sebazinsäure, eine 1,12-C₁₂-Dicarbonsäure, Brassylsäure, 1,16-C₁₆-Dicarbonsäure, 1,18-C₁₈-Dicarbonsäure oder 1,36-C₃₆-Dicarbonsäure oder Gemische dieser Dicarbonsäuren in Frage. Bevorzugt sind Bernsteinsäure, Glutarsäure, Adipinsäure, Azelainsäure, Sebazinsäure oder eine 1,12-C₁₂-Dicarbonsäure oder Mischungen davon und insbesondere bevorzugt die Bernsteinsäure, Adipinsäure, oder Sebazinsäure oder Mischungen davon.

Als sulfonatgruppenhaltige Dicarbonsäure (Komponente a3) kommen insbesondere aromatische Sufonsäuren bzw. deren Salze und insbedondere bevorzugt deren Alkalisalze infrage. Geeignet sind beispielsweise die 1,4-Benzoldicarbonsäure-2 sulfonsäure, 1,3-Benzoldicarbonsäure-5 sulfonsäure - im Folgenden auch Isophthalsäure-5 sulfonsäure genannt -, 1,2-Benzoldicarbonsäure-3 sulfonsäure, 1,2-Benzoldicarbonsäure-4 sulfonsäure bzw. deren Salze und insbedondere bevorzugt deren Alkalisalze. Besonders bevorzugt ist Isophthalsäure-5 sulfonsäure Natriumsalz (auch verkürzt NaSiP genannt).

Sowohl von den zuvor beschriebenen aromatischen Polyestern wie den aliphatisch-aromatischen Polyestern sind diejenigen bevorzugt, die 10 bis 60 mol-%, vorzugsweise 15 bis 40 mol-%, bezogen auf die Komponenten a1) bis a3), einer sulfonatgruppenhaltigen Dicarbonsäure und besonders bevorzugt Isophthalsäure-5-sulfonsäure Natriumsalz enthalten, da diese geeignet sind, um wässrige Lösungen zu bilden.

Als Diole (Komponente b1) kommen aliphatische C₂-C₁₂-Diole wie Ethylenglykol, 1,2-Propandiol, 1,3-Propandiol, 1,2-Butandiol, 1,4-Butandiol, 1,5-Pentandiol, 2,4-Dimethyl-2-ethylhexan-1,3-diol, 2,2-Dimethyl- 1,3-propandiol, 2-Ethyl-2-butyl-1,3-propandiol, 2-Ethyl-2-isobutyl- 1,3-propandiol, 1,6-Hexandiol, 1,8-Oktandiol und 2,2,4-Trimethyl-1,6-hexandiol in Frage, wobei Ethylenglykol, 1,3-Propandiol, 1,4-Butandiol und 2,2-Dimethyl- 1,3-propandiol (Neopentylglykol) bevorzugt sind. Letztere haben zudem den Vorteil, dass sie als nachwachsender Rohstoff zugänglich sind. Es können auch Mischungen unterschiedlicher Alkandiole verwendet werden.

Bei den erfindungsgemäßen aromatischen Polyestern sind diejenigen bevorzugt, die Ethylenglykol, oder Cyclohexandimethanol als Diolkomponente oder anstelle von b1) Diethylenglylol oder Triethylenglykol als Komponente b2) enthalten und bei den erfindungsgemäßen aliphatisch-aromatischen Polyestern sind insbesondere diejenigen bevorzugt, die 1,4-Butandiol als Diolkomponente enthalten.

Als Diole (Komponente b1) kommen auch cycloaliphatische C₆-C₁₂-Diole wie Cyclopentandiol, 1,4-Cyclohexandiol, 1,4-Cyclohexandimethanol (cis/trans), 1,4-Di(hydroxymethyl)cyclohexan oder 2,5-Tetrahydrofurandimethanol in Frage, wobei 1,4-Cyclohexandimethanol bevorzugt ist.

In einer Ausführungsform enthalten die erfindungsgemäßen Polyester Als Komponente E 0 bis 4 Gew% , vorzugsweise 0,01 bis 1,0 Gew% und insbesondere bevorzugt 0,05 bis 0,3 Gew%, bezogen auf die Summe der Komponenten A und B und C, eines mindestens 3 funktionelle Gruppen enthaltenden Verzweigers (Komponente E). Der Verzweiger ist bevorzugt ein mindestens trifunktioneller Alkohol oder eine mindestens trifunktionelle Carbonsäure.

Besonders bevorzugte Verzweiger haben drei bis sechs funktionelle Gruppen. Beispielhaft seien genannt: Weinsäure, Zitronensäure, Äpfelsäure; Trimethylolpropan, Trimethylolethan; Pentaerythrit; Polyethertriole und Glycerin, Trimesinsäure, Trimellitsäure, Trimellitsäureanhydrid, Pyromellitsäure und Pyromellitsäuredianhydrid. Bevorzugt sind Polyole wie Trimethylolpropan, Pentaerythrit und insbesondere Glycerin. Mittels der Komponente lassen sich biologisch abbaubare Polyester mit einer strukturellen Viskosität aufbauen. Die biologisch abbaubaren Polyester lassen sich leichter verarbeiten.

Die Komponente b2 besteht aus einer Etherfunktionen enthaltenden Dihydroxyverbindung der Formel I

HO-[(CH₂)ₙ-O]ₘ-H (I)

in der n für 2, 3 oder 4 und m für eine ganze Zahl von 2 bis 250 stehen,

Als Dihydroxyverbindungen eignen sich Diethylenglykol, Triethylenglykol, Polyethylenglykol, Polypropylenglykol und Polytetrahydrofuran (Poly-THF), besonders bevorzugt Diethylenglykol, Triethylenglykol und Polyethylenglykol, ein, wobei man auch Mischungen davon oder Verbindungen, die unterschiedliche Variablen n aufweisen (siehe Formel I), beispielsweise Polyethylenglykol, das Propyleneinheiten (n = 3) enthält, beispielsweise erhältlich durch Polymerisation nach an sich bekannten Methoden von zuerst Ethylenoxid und anschließend mit Propylenoxid, besonders bevorzugt ein Polymer auf Basis von Polyethylenglykol, mit unterschiedlichen Variablen n, wobei Einheiten gebildet aus Ethylenoxid überwiegen. Das Molekulargewicht (Mₙ) des Polyethylenglykols wählt man in der Regel im Bereich von 250 bis 8000, bevorzugt von 600 bis 3000 g/mol.

Als Hydroxycarbonsäure c1) kann eingesetzt werden: Glykolsäure, D-, L-, D,L-Milchsäure, 6-Hydroxyhexansäure, deren cyclische Derivate wie Glycolid (1,4-Dioxan-2,5-dion), D-, L-Dilactid (3,6-dimethyl-1,4- dioxan-2,5-dion), p-Hydroxybenzoesäure sowie deren Oligomere und Polymere wie 3-Polyhydroxybuttersäure, Polyhydroxyvaleriansäure, Polymilchsäure (wie beispielsweise die unter dem Markennamen Ingeo® vertriebenen Produkte der Fa. NatureWorks) sowie eine Mischung aus 3-Polyhydroxybuttersäure und 4-Polyhydroxybutyraten oder 3-Polyhydroxy-valeriansäure oder 3-Polyhydroxy-hexansäure besonders bevorzugt für die Herstellung von Polyestern sind die niedermolekularen und cyclischen Derivate davon.

Die Hydroxycarbonsäuren können beispielsweise in Mengen von 0,01 bis 50, bevorzugt von 0,1 bis 30 Gew.-% bezogen auf die Menge an A und B verwendet werden.

Als Amino-C₂-C₁₂-alkanol oder Amino-C₅-C₁₀-cyloalkanol (Komponente b3), wobei hierunter auch 4-Aminomethylcyclohexanmethanol fallen soll, setzt man bevorzugt Amino-C₂-C₆-alkanole wie 2-Aminoethanol, 3-Aminopropanol, 4-Aminobutanol, 5-Aminopen-tanol, 6-Aminohexanol sowie Amino-C₅-C₆-cyloalkanole wie Aminocyclopentanol und Aminocyclohexanol oder Mischungen davon ein.

Als Diamino-C₁-C₈-alkan (Komponente b4) setzt man bevorzugt Diamino-C₄-C₆-alkane ein wie 1,4-Diminobutan, 1,5-Diaminopentan und 1,6-Diaminohexan (Hexamethylendiamin, "HMD").

Bevorzugt ist c2 ist eine Aminocarbonsäure ausgewählt aus der Gruppe, bestehend aus Caprolactam, 1,6 Aminocapronsäure, Laurinlactam, 1,12-Aminolaurinsäure und 1,11-Aminoundecansäure.

Als Komponente c2 können Aminocarbonsäuren ausgewählt aus der Gruppe, bestehend aus Caprolactam, 1,6 Aminocapronsäure, Laurinlactam, 1,12-Aminolaurinsäure und 1,11-Aminoundecansäure verwendet werden.

Im Allgemeinen wird c2 in Mengen von 0 bis 20, bevorzugt von 0,1 bis 10 Gew.-%, bezogen auf die Gesamtmenge der Komponenten A und B, eingesetzt.

Als Komponente D werden in der Regel ein Isocyanat oder Isocyanurat oder eine Mischung unterschiedlicher Isocyanate und Isocyanurate eingesetzt. Als Isocyanate können aromatische oder aliphatische Diisocyanate eingesetzt werden. Es können aber auch höher funktionelle Isocyanate verwendet werden.

Unter einem aromatischen Diisocyanat werden im Rahmen der vorliegenden Erfindung vor allem Toluylen-2,4-diisocyanat, Toluylen-2,6-diisocyanat, 2,2'-Diphenylmethandiisocyanat, 2,4'-Diphenylmethandiisocyanat, 4,4'-Diphenylmethan-diisocyanat, Naphthylen-1,5-diisocyanat oder Xylylen-diisocyanat verstanden.

Darunter werden 2,2'-, 2,4'- sowie 4,4'-Diphenylmethandiisocyanat besonders bevorzugt. Im Allgemeinen werden letztere Diisocyanate als Mischung eingesetzt.

Als dreikerniges Isocyanat kommt auch Tri(4-isocyanophenyl)methan in Betracht. Die mehrkernigen aromatischen Diisocyanate fallen beispielsweise bei der Herstellung von ein- oder zweikernigen Diisocyanaten an.

In untergeordneten Mengen, z.B. bis zu 5 Gew.-%, bezogen auf das Gesamtgewicht der Komponente D, kann die Komponente d1 auch Urethiongruppen, beispielsweise zum Verkappen der Isocyanatgruppen, enthalten.

Unter einem aliphatischen Diisocyanat D werden im Rahmen der vorliegenden Erfindung vor allem lineare oder verzweigte Alkylendiisocyanate oder Cycloalkylendiisocyanate mit 2 bis 20 Kohlenstoffatomen, bevorzugt 3 bis 15 Kohlenstoffatomen, z.B. 1,6-Hexamethylendiisocyanat, Isophorondiisocyanat oder Methylen-bis(4-isocyanatocyclo-hexan), verstanden. Besonders bevorzugte aliphatische Diisocyanate D sind Isophorondiisocyanat und insbesondere 1,6-Hexamethylendiisocyanat.

Zu den bevorzugten Isocyanuraten zählen die aliphatischen Isocyanurate, die sich von Alkylendiisocyanaten oder Cycloalkylendiisocyanaten mit 2 bis 20 Kohlenstoffatomen, bevorzugt 3 bis 15 Kohlenstoffatomen, z.B. Isophorondiisocyanat oder Methylen-bis(4-isocyanatocyclohexan), ableiten. Dabei können die Alkylendiisocyanate sowohl linear als auch verzweigt sein. Besonders bevorzugt werden Isocyanurate, die auf n-Hexamethylendiisocyanat basieren, beispielsweise cyclische Trimere, Pentamere oder höhere Oligomere des 1,6-Hexamethylendiisocyanats.

Im allgemeinen wird die Komponente D in Mengen von 0,1 bis 4, bevorzugt 0,2 bis 1,2 Gew.-%, bezogen auf die Komponenten A und B und gegebenenfalls C, eingesetzt.

Erfindungsgemäße aromatische Polyester können enthalten 100 mol-%, bezogen auf die Komponenten a1) und a2), 2,5-Furandicarbonsäure und damit keine Komponente a2). Beispielsweise umfassen aromatische Polyester: Poly(alkylen-2,5-furandicarboxylate) wie Poly(ethylen-2,5-furandicarboxylat), Poly(propylen-2,5-furandicarboxylat), Poly(butylen-2,5-furandicarboxylat), Poly(hexylen-2,5-furandicarboxylat), Poly(octylen-2,5-furandicarboxylat), oder Poly(alkoxylen-2,5-furandicarboxylate) wie Poly(ethoxyethylen-2,5-furandicarboxylate) oder Poly(diethoxyethylen-2,5-furandicarboxylate).

Erfindungsgemäße aliphatisch-aromatische Polyester enthalten bevorzugt 25 bis 99 mol-%, vorzugsweise 60 bis 75 mol-% und insbesondere bevorzugt 65 bis 75 mol-% einer 2,5-Furandicarbonsäure und entsprechend 1 bis 75 mol-%, vorzugsweise 25 bis 40 mol-% und insbesondere bevorzugt 25 bis 35 mol-% einer aliphatischen C₄-C₃₆-Dicarbonsäure bezogen auf die Summe von Komponente a1) und a2). Aliphatisch-aromatische Polyester umfassen insbesondere: Poly-butylen-2,5-furandicarboxylat-coadipat, Poly-butylen-2,5-furandicarboxylat-coazelat, Poly-butylen-2,5-furandicarboxylat-cosebacat, Poly-butylen-2,5-furandicarboxylat-cobrassylat, Poly-butylen-2,5-furandicarboxylat-co-1,12-C₁₂-dicarboxylat, Poly-butylen-2,5-furandicarboxylat-co-1,18-C₁₈-dicarboxylat und Poly-butylen-2,5-furandicarboxylat-co-1,36-C₃₆-dicarboxylat.

Die Viskositätszahl der erfindungsgemäßen Polyester gemäß EN-ISO 1628-1:2012-10 (gemessen in 0,05 g/ml Lösung Phenol/o-Dichlorbenzol (1:1)) liegt bevorzugt zwischen 50 und 450, vorzugsweise von 70 bis 250 mL/g (gemessen in o-Dichlorbenzol/Phenol (Gewichtsverhältnis 50/50). Der Schmelzpunkt liegt bevorzugt im Bereich von 85 bis 150, besonders bevorzugt im Bereich von 95 bis 140°C.

Im Sinne der vorliegenden Erfindung ist das Merkmal "biologisch abbaubar" für einen Stoff oder ein Stoffgemisch dann erfüllt, wenn dieser Stoff oder das Stoffgemisch entsprechend DIN EN 13432 einen prozentualen Grad des biologischen Abbaus von mindestens 90% aufweist.

Im Allgemeinen führt die biologische Abbaubarkeit dazu, dass die Polyester(mischungen) in einer angemessenen und nachweisbaren Zeitspanne zerfallen. Der Abbau kann enzymatisch, hydrolytisch, oxidativ und/oder durch Einwirkung elektromagnetischer Strahlung, beispielsweise UV-Strahlung, erfolgen und meist zum überwiegenden Teil durch die Einwirkung von Mikroorganismen wie Bakterien, Hefen, Pilzen und Algen bewirkt werden. Die biologische Abbaubarkeit lässt sich beispielsweise dadurch quantifizieren, dass Polyester mit Kompost gemischt und für eine bestimmte Zeit gelagert werden. Beispielsweise wird gemäß DIN EN 13432 CO₂-freie Luft durch gereiften Kompost während des Kompostierens strömen gelassen und dieser einem definierten Temperaturprogramm unterworfen. Hierbei wird die biologische Abbaubarkeit über das Verhältnis der Netto-CO₂-Freisetzung der Probe (nach Abzug der CO₂-Freisetzung durch den Kompost ohne Probe) zur maximalen CO₂-Freisetzung der Probe (berechnet aus dem Kohlenstoffgehalt der Probe) als prozentualer Grad des biologischen Abbaus definiert. Biologisch abbaubare Polyester(mischungen) zeigen in der Regel schon nach wenigen Tagen der Kompostierung deutliche Abbauerscheinungen wie Pilzbewuchs, Riss- und Lochbildung.

Andere Methoden zur Bestimmung der Bioabbaubarkeit werden beispielsweise in ASTM D 5338 und ASTM D 6400 und OECD 301 beschrieben.

Die erfindungsgemäßen Polyester und Polyestermischungen können bevorzugt die folgenden Füllstoffe enthalten.

Calciumcarbonat kann beispielsweise in 10 bis 25 Gew.-%, bevorzugt 10 bis 20 Gew.-%, besonders bevorzugt 12 bis 28 Gew.-%, bezogen auf das Gesamtgewicht der Polymermischung, eingesetzt werden. Unter anderem hat sich das Calciumcarbonat der Fa. Omya als geeignet erwiesen. Das Calciumcarbonat weist in der Regel eine mittlere Teilchengröße von 0,5 bis 10 Mikrometern bevorzugt 1 - 5, besonders bevorzugt 1 - 2,5 Mikrometern auf.

Talk kann beispielsweise in 3 bis 15 Gew.-%, bevorzugt 5 bis 10 Gew.-%, besonders bevorzugt 5 bis 8 Gew.-%, bezogen auf das Gesamtgewicht der Polymermischung, eingesetzt werden. Unter anderem hat sich der Talk der Fa. Mondo Minerals als geeignet erwiesen. Der Talk weist in der Regel eine mittlere Teilchengröße von 0,5 - 10, bevorzugt 1 - 8, besonders bevorzugt 1 - 3 Mikrometern auf.

Neben den Füllstoffen Calciumcarbonat und Talk können noch weitere Mineralien enthalten sein wie: Graphit, Gips, Ruß, Eisenoxid, Calciumchlorid, Kaolin, Siliziumdioxid (Quarz), Natriumcarbonat, Titandioxid, Silikat, Wollastonit, Glimmer, Montmorellonite, Mineralfasern und Naturfasern.

Naturfasern sind in der Regel Cellulosefasern, Kenaffasern, Hanffasern, Holzmehl oder Kartoffelschalen. Sie werden vorzugsweise in 1 bis 20 Gew.-% bezogen auf die Polymermischung eingesetzt.

Die Minerialien inklusive der Füllstoffe Calciumcarbonat und Talk können auch als Nanofüllstoffe eingesetzt werden. Nanofüllstoffe sind insbesondere feinteilige Schichtsilikate, bevorzugt Tonmineralien, besonders bevorzugt Montmorillonit enthaltend Tonmineralien, deren Oberfläche mit einem oder mehreren quarternären Ammoniumsalzen und/oder Phosphoniumsalzen und/oder Sulfoniumsalzen modifiziert sind. Als Tonmineralien bevorzugt sind natürliche Montmorillonite und Bentonite.

Insgesamt können den Polyestermischungen beispielsweise Füllstoffe in 10 bis 35 Gew.-%, bezogen auf das Gesamtgewicht der Polymermischung, zugesetzt werden.

Die erfindungsgemäßen Polyester können verwendet werden zur Festigung von Haaren, auch Hair Styling genannt. Dazu können die erfindungsgemäßen Polyester als Bestandteil von Formulierungen eingesetzt werden, die im Übrigen dem Fachmann bekannte Inhaltsstoffe enthalten, die für Hair Styling Formulierungen geeignet sind.

### Beispiele

### Bezeichnungen, Abkürzungen und molare Massen M (in g/mol)

- 2,5-FDCS: 2,5-Furandicarbonsäure
- 2,5-FDCS-DME: 2,5-Furandicarbonsäuredimethylester, M = 184 g/mol (auch: Dimethyfurandicarbonsäure)
- NaSIP: Natriumsulfoisophthalsäure; Strukturformel: 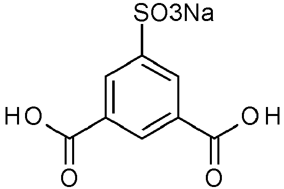
- DMNaSIP: Natriumsulfoisophthalsäuredimethylester; M = 296 g/mol (auch: Dimethylnatriumsulfoisophthalsäure)
- HDI: Hexamethylendiisocyanat
- EG: Ethylenglykol, M = 62 g/mol
- DEG: Diethylenglykol; M = 106 g/mol
- BD: 1,4-Butandiol; M = 90 g/mol
- 1,8-Octandiol: M = 146 g/mol
- Triethylenglykol: M = 150 g/mol
- Tetraethylenglykol: M = 194 g/mol

### Prüfmethoden

Die Eigenschaften der gemäß der Beispiele hergestellten Polyester wurden mit folgenden Methoden geprüft. Wenn dabei auf Normen verwiesen wird, danni ist jeweils die Version der Norm gemeint, die am 1. August 2016 in Kraft war.

Die Bestimmung der Viskositätszahlen VZ erfolgte nach EN-ISO 1628-1:2012-10, Kapillarviskosimetrie. Zum Einsatz kam ein Mikro-Ubbelohde Viskosimeter, Typ M-II. Als Lösungsmittel wurde das Gemisch: Phenol/o-Dichlorbenzol im Gewichtsverhältnis 50/50 verwendet.

Der E-Modul und die Streckspannung wurde mittels eines Zugversuchs an Zugstäben mit einer Dicke von etwa 420 µm gemäß ISO 527-3: 2003 bestimmt.

Die Glastemperatur Tg wurde nach DIN EN ISO 11357-3:2913-04 gemessen.

### Allgemeine Herstellmethode der Polyester

Im Folgenden sind die Angaben in mol-% bezogen auf die Summe der Mole an Dimethyfurandicarbonsäure und Dimethylnatriumsulfoisophthalsäure, welche als 100 mol-% angesetzt wird.

Zur Herstellung der Polyester wurden 70 mol-% 2,5-Dimethyfurandicarbonsäure, 30 mol-% Dimethylnatriumsulfoisophthalsäure, 130 mol-% des jeweils angegebenen Diols und ggf. die angegebene Menge Glycerin zusammen mit der angegebenen Menge Tetrabutylorthotitanat (TBOT) gemischt. Das Reaktionsgemisch wurde auf eine Temperatur von 180°C erhitzt und bei dieser Temperatur 2 h lang umgesetzt. Anschließend wurde die Temperatur auf 240°C erhöht und das überschüssige Diol unter Vakuum über einen Zeitraum von 1 - 2 h abdestilliert. Anschließend wurde ggf. bei 240°C die angegebene Menge Hexamethylendiisocyanat innerhalb von10 min langsam zugegeben.

Bei diesem Verfahren wirkt das TBOT als Katalysator der Polykondensation. Glycerin wirkt als Verzweiger und HDI wirkt als "Kettenverlängerer". Bei der Zugabe von HDI reagieren die vorhandenen Polyesterketten über ihre OH-Endgruppen mit dem HDI und bilden Urethangruppen. Dadurch wird die mittlere molare Masse des Polyesters erhöht.

### Verfahren zur Herstellung der Polyester gemäß der Beispiele

Entsprechende der allgemeinen Herstellmethode der Polyester wurden Polyester hergestellt.

Dabei wurden die folgenden Stoffe eingesetzt. Die erhaltenen Polyester hatten die angegebenen Eigenschaften (Tg, VZ, E-Modul, Streckspannung). Die Mengen an Glycerin und HDI sind in g und in Gew.-% angegeben, wobei sich die Gew.-% beziehen auf die Summe der Massen der Wiederholungseinheiten abgeleitet von 2,5-FDCS, NaSIP und dem verwendeten Diol, die zu 100 Gew.-% gesetzt wurde.

### Beispiel 1-1

| | |
|---|---|
| 2,5-FDCS-DME: | 64,3 g (0,35 mol) (70 mol-%) |
| DMNaSIP: | 44,3 g (0,15 mol) (30 mol-%) |
| Ethylenglykol: | 40,3 g (0,65 mol) (130 mol-%) |
| Glycerin: | 0,11 g (0,1 Gew.-%) (0,0012 mol) |
| TBOT: | 0,08 g |
| HDI: | 0,86 g (0,8 Gew.-%) (0,0051 mol) |

Tg: 84°C, VZ: 80, E-Modul: 2950 MPa, Streckspannung: 65 MPa

### Beispiel 1-2

| | |
|---|---|
| 2,5-FDCS-DME: | 64,3 g (0,35 mol) (70 mol-%) |
| DMNaSIP: | 44,3 g (0,15 mol) (30 mol-%) |
| Ethylenglykol: | 40,3 g (0,65 mol) (130 mol-%) |
| TBOT: | 0,08 g |
| HDI: | 0,86 g (0,8 Gew.-%) |

Tg: 82°C, VZ: 76, E-Modul: 2900 MPa, Streckspannung: 60 MPa

### V-Beispiel 1

Das ist ein Vergleichsbeispiel. Es ist nicht erfindungsgemäß, weil es weder HDI noch Glycerin enthält.

| | |
|---|---|
| 2,5-FDCS-DME: | 64,3 g (0,35 mol) (70 mol-%) |
| DMNaSIP: | 44,3 g (0,15 mol) (30 mol%) |
| Ethylenglykol: | 40,3 g (0,65 mol) (130 mol%) |
| TBOT: | 0,08 g |

Tg: 80°C, VZ: 35, E-Modul: 2900 MPa, Streckspannung: 35 MPa

### Beispiel 2

| | |
|---|---|
| 2,5-FDCS-DME: | 54,0 g (0,293 mol) (70 mol-%) |
| DMNaSIP: | 37,2 g (0,125 mol) (30 mol%) |
| Diethylenglykol: | 57,8 g (0,545 mol) (130 mol%) |
| Glycerin: | 0,11 g (0,1 Gew.-%) |
| TBOT: | 0,08 g |
| HDI: | 0,87 g (0,8 Gew.-%) |

Tg: 61°C, VZ: 85

### V-Beispiel 2

Das ist ein Vergleichsbeispiel. Es ist nicht erfindungsgemäß, weil es weder HDI noch Glycerin enthält.

| | |
|---|---|
| 2,5-FDCS-DME: | 54,0 g (0,293 mol) (70 mol-%) |
| DMNaSIP: | 37,2 g (0,125 mol) (30 mol%) |
| Diethylenglykol: | 57,8 g (0,545 mol) (130 mol%) |
| TBOT: | 0,08 g |

Tg: 61°C, VZ: 43

### Beispiel 3

| | |
|---|---|
| 2,5-FDCS-DME: | 57,3 g (0,31 mol) (70 mol-%) |
| DMNaSIP: | 39,5 g (0,13 mol) (30 mol%) |
| 1,4-Butandiol: | 52,1 g (0,58 mol) (130 mol%) |
| Glycerin: | 0,11 g (0,1 Gew.-%) |
| TBOT: | 0,08 g |
| HDI: | 0,87 g (0,8 Gew.-%) |

Tg: 57°C, VZ: 73

### Beispiel 4

| | |
|---|---|
| 2,5-FDCS-DME: | 47,1 g (0,256 mol) (70 mol-%) |
| DMNaSIP: | 32,5 g (0,11 mol) (30 mol%) |
| 1,8-Octandiol: | 69,4 g (0,475 mol) (130 mol%) |
| Glycerin: | 0,11 g (0,1 Gew.-%) |
| TBOT: | 0,08 g |
| HDI: | 0,88 g (0,8 Gew.-%) |

Tg: 6°C, VZ: 83

### Beispiel 5

| | |
|---|---|
| 2,5-FDCS-DME: | 46,5 g (0,253 mol) (70 mol-%) |
| DMNaSIP: | 32,1 g (0,108 mol) (30 mol%) |
| Triethylenglykol: | 70,4 g (0,469 mol) (130 mol%) |
| Glycerin: | 0,11 g (0,1 Gew.-%) |
| TBOT: | 0,08 g |
| HDI: | 0,88 g (0,8 Gew.-%) |

Tg: 29°C, VZ: 78

### Beispiel 6

| | |
|---|---|
| 2,5-FDCS-DME: | 40,8 g (0,2217 mol) (70 mol-%) |
| DMNaSIP: | 28,1 g (0,095 mol) (30 mol%) |
| Tetraethylenglykol: | 80,0 g (0,41 mol) (130 mol%) |
| Glycerin: | 0,11 g (0,1 Gew.-%) |
| TBOT: | 0,08 g |
| HDI: | 0,88 g (0,8 Gew.-%) |

Tg: 14°C, VZ: 75

### Zusammensetzung der hergestellten Polyester

Die Angaben in mol-% sind bezogen auf die Summe aus Wiederholungseinheiten abgeleitet von 2,5-FDCS und von Na-SIP. Diese Summe wurde als 100 mol-% angesetzt. Alle Polyester enthielten:
- 70 mol-% Wiederholungseinheiten abgeleitet von 2,5-FDCS
- 30 mol-% Wiederholungseinheiten abgeleitet von NaSIP
- 100 mol-% Wiederholungseinheiten abgeleitet von dem jeweiligen Diol (der Überschuß der anfänglich vorhandenen 130 mol-% wurde abdestilliert)
- Soweit vorhanden außerdem Wiederholungseinheiten abgeleitet von Glycerin und/oder Wiederholungseinheiten abgeleitet von HDI. Die Wiederholungseinheiten abgeleitet von Glycerin gehen dabei auf Kosten der Wiederholungseinheiten abgeleitet von dem verwendeten Diol, dessen Anteil im Polyester also genau genommen etwas unter 100 mol-% liegt, wenn Glycerin eingesetzt wird. Da die eingesetzte Menge Glycerin gering war, wurde das bei den Angaben zur Menge der Wiederholungseinheiten abgeleitet vom Diol nicht berücksichtigt

Angaben in mol-%, wenn nicht Gew.-% angegeben sind; B = Beispiel; VB = Vergleichsbeispiel

| Wiederholungseinheiten abgeleitet von: | B 1-1 (mol-%) | B 1-2 | VB 1 | B 2 |
|---|---|---|---|---|
| 2,5-FDCS | 70 | 70 | 70 | 70 |
| NaSIP | 30 | 30 | 30 | 30 |
| 1,4-Butandiol | - | - | - | - |
| Ethylenglycol | 100 | 100 | 100 | |
| Diethylenglycol | - | - | - | 100 |
| Triethylenglycol | - | - | - | - |
| Tetraethylenglycol | - | - | - | - |
| 1,8-Octandiol | - | - | - | - |
| Glycerin | 0,1 Gew.-% | - | - | 0,1 Gew.-% |
| HDI | 0,8 Gew.-% | 0,8 Gew.-% | - | 0,8 Gew.-% |

| Wiederholungseinheiten abgeleitet von: | VB 2 (mol-%) | B 3 | B 4 | B 5 |
|---|---|---|---|---|
| 2,5-FDCS | 70 | 70 | 70 | 70 |
| NaSIP | 30 | 30 | 30 | 30 |
| 1,4-Butandiol | - | 100 | - | - |
| Ethylenglycol | - | - | - | - |
| Diethylenglycol | 100 | - | - | - |
| Triethylenglycol | - | - | - | 100 |
| Tetraethylenglycol | - | - | - | - |
| 1,8-Octandiol | - | - | 100 | - |
| Glycerin | - | 0,1 Gew.-% | 0,1 Gew.-% | 0,1 Gew.-% |
| HDI | - | 0,8 Gew.-% | 0,8 Gew.-% | 0,8 Gew.-% |

| Wiederholungseinheiten abgeleitet von: | B 6 (mol-%) |
|---|---|
| 2,5-FDCS | 70 |
| NaSIP | 30 |
| 1,4-Butandiol | - |
| Ethylenglycol | - |
| Diethylenglycol | - |
| Triethylenglycol | - |
| Tetraethylenglycol | 100 |
| 1,8-Octandiol | - |
| Glycerin | 0,1 Gew.-% |
| HDI | 0,8 Gew.-% |

Die Glasübergangstemperatur Tg der Polyester ist offensichtlich abhängig von dem verwendeten Diol.

Die Werte für VZ (Viskositätszahl, ein Maß für die mittlere molare Masse (Gewichtsmittel) der Polyester) und für die Streckspannung liegen bei den Vergleichsbeispielen deutlich niedriger als bei den erfindungsgemäßen Beispielen. Die Polyester der erfindungsgemäßen Beispiele haben also eine höhere mittlere molare Masse (Gewichtsmittel) Mw und eine höhere mechanische Festigkeit.

## Patentansprüche

1. Ein Polyester bestehend aus:
A) Wiederholungseinheiten abgeleitet von einer Säurekomponente, welche besteht aus
a1) 30 bis 90 mol-%, bezogen auf die Summe der Komponenten a1, a2 und a3, 2,5-Furandicarbonsäure,
a2) 0 bis 60 mol-%, bevorzugt 0 bis 50 mol-%, bezogen auf die die Summe der Komponenten a1, a2 und a3, einer aliphatischen C₄ -C₃₆-Dicarbonsäure oder eine Mischung aus mehreren aliphatischen C₄ -C₃₆-Dicarbonsäuren, und
a3) 10 bis 60 mol-%, bezogen auf die Summe der Komponenten a1, a2 und a3, einer sulfonatgruppenhaltigen Dicarbonsäure,
wobei die Molprozente der Komponenten a1) bis a3) zusammen 100 mol-% ergeben,
wobei bevorzugt der Anteil von a1 55 bis 85 mol-% und gleichzeitig der Anteil von a2 0 bis 30 mol-% ist und gleichzeitig der Anteil von a3 15 bis 45 mol-% ist,
B) Wiederholungseinheiten abgeleitet von einer Di-ol/amin-komponente bestehend aus
b1) einem C₂-bis C₁₂-Alkandiol oder einer Mischung aus mehreren C₂-bis C₁₂-Alkandiolen, und/oder
b2) einer Dihydroxyverbindung der Formel I,
HO-[(CH₂)ₙ-O]ₘ-H (I)
in der n für 2, 3 oder 4 und m für eine ganze Zahl von 2 bis 250 stehen, oder einer Mischung mehrerer solcher Dihydroxyverbindungen,
b3) optional einem oder mehreren Amino-C₂- bis C₁₂-alkanolen oder einem oder mehreren Amino-C₅-bis C₁₀-cycloalkanolen oder Mischungen davon, und
b4) optional einem oder mehreren Diamino-C₁- bis C₈-Alkanen,
wobei die Menge an B so gewählt ist, dass pro 100 mol A 100 mol B vorhanden sind, verringert um die Menge an Molen an D und E,
und wobei die Summe der Anteile von b1 und von b2 an B mindestens 50 mol-%, bevorzugt mindestens 80 mol-%, ist,
C) optional Wiederholungseinheiten abgeleitet von
c1) einer oder mehreren Hydroxycarbonsäuren der Formel IIa,
HO-C(O)-G-OH (IIa)
in der G für einen Rest steht, der ausgewählt ist aus der Gruppe bestehend aus Phenylen, -(CH₂)q-, wobei q eine ganze Zahl von 1 bis 5 bedeutet, - C(R)H- und -C(R)HCH₂, wobei R für Methyl oder Ethyl steht, und/oder
c2) einer oder mehreren Aminocarbonsäuren oder deren cylischen Amiden ausgewählt aus der Gruppe bestehend aus Caprolactam, 1,6-Aminocapronsäure, Laurinlactam, 1,12-Aminolaurinsäure und 1,11-Aminoundecansäure,
wobei bevorzugt der Anteil von C an der Gesamtmasse des Polyesters maximal 20 Gew.-%, insbesondere bevorzugt maximal 10 Gew.-% ist,
D) 0,1 bis 4 Gew.-%, bezogen auf die Summe der Komponenten A und B und C, Wiederholungseinheiten abgeleitet von mindestens einer di- oder oligofunktionellen Verbindung ausgewählt aus der Gruppe bestehend aus einem Di- oder Oligo-Isocyanat und einem Di- oder Oligo-Isocyanurat, und
E) 0 bis 4 Gew.-%, bezogen auf die Summe der Komponenten A und B und C, Wiederholungseinheiten abgeleitet von einem Verzweiger, der mindestens drei funktionelle Gruppen enthält, wobei die funktionellen Gruppen ausgewählt sind aus der Gruppe bestehend aus COOH, OH, NH2 und Mischungen davon.

2. Der Polyester nach Anspruch 1, wobei die Summe der Anteile a2, b3, b4, c1 und c2, bezogen auf die gesamte Masse des Polyesters, maximal 10 Gew.-%, bevorzugt maximal 5 Gew.-% und insbesondere bevorzugt 0 Gew.-% ist.

3. Der Polyester nach Anspruch 1 oder 2, wobei a3 eine aromatische sulfonatgruppenhaltigen Dicarbonsäure ist, in der die Sulfonatgruppe entweder als freie Säure oder als Salz, bevorzugt als Alkalisalz und insbesondere als Natriumsalz vorliegt.

4. Der Polyester nach Anspruch 3, wobei a3 ausgewählt ist aus der Gruppe bestehend aus 1,4-Benzoldicarbonsäure-2-sulfonsäure, 1,3-Benzoldicarbonsäure-5-sulfonsäure (auch Isophthalsäure-5-sulfonsäure genannt), 1,2-Benzoldicarbonsäure-3-sulfonsäure und 1,2-Benzoldicarbonsäure-4-sulfonsäure, jeweils in der Form der freien Säure oder eines Salzes, bevorzug in der Form eines Alkalisalzes, insbesondere in der Form des Natriumsalzes, wobei Mononatrium-Isophthalsäure-5-sulfonsäure besonders bevorzugt ist.

5. Der Polyester nach einem der Ansprüche 1 bis 4, wobei b1 ausgewählt ist aus der Gruppe bestehend aus Ethylenglykol, 1,2-Propandiol, 1,3-Propandiol, 1,2-Butandiol, 1,4-Butandiol, 1,5-Pentandiol, 2,4-Dimethyl-2-ethylhexan-1,3-diol, 2,2-Dimethyl-1,3-propandiol, 2-Ethyl-2-butyl-1,3-propandiol, 2-Ethyl-2-isobutyl-1,3-propandiol, 1,6-Hexandiol, 1,8-Oktandiol und 2,2,4-Trimethyl-1,6-hexandiol, wobei b1 bevorzugt ausgewählt ist aus der Gruppe bestehend aus Ethylenglykol, 1,4-Butandiol, 1,6-Hexandiol und 1,8-Oktandiol .

6. Der Polyester nach einem der Ansprüche 1 bis 5, wobei b2 ausgewählt ist aus der Gruppe bestehend aus Diethylenglykol, Triethylenglykol, Tetraethylenglykol, Polyethylenglykol, Polypropylenglykol und Polytetrahydrofuran, wobei b2 bevorzugt ausgewählt ist aus der Gruppe bestehend aus Diethylenglykol, Triethylenglykol und Tetraethylenglykol.

7. Der Polyester nach einem der Ansprüche 1 bis 6, wobei D Wiederholungseinheiten sind abgeleitet von einem aliphatischen Diisocyanat, wobei das Diisocyanat bevorzugt ein lineares oder verzweigtes Alkylendiisocyanat oder Cycloalkylendiisocyanat, mit in beiden Fällen 2 bis 20, insbesondere 3 bis 15, Kohlenstoffatomen ist, und wobei D insbesondere bevorzugt 1,6-Hexamethylendiisocyanat ist.

8. Der Polyester nach einem der Ansprüche 1 bis 7, wobei E Wiederholungseinheiten sind abgeleitet von einem Verzweiger ausgewählt aus der Gruppe bestehend aus Weinsäure, Zitronensäure, Äpfelsäure, Trimethylolpropan, Trimethylolethan, Pentaerythrit, einem Polyethertriol, Glycerin, Trimesinsäure, Trimellitsäure und Pyromellitsäure, wobei E bevorzugt Wiederholungseinheiten sind abgeleitet von Trimethylolpropan, Pentaerythrit oder Glycerin, und wobei E insbesondere bevorzugt Wiederholungseinheiten sind abgeleitet von Glycerin.

9. Eine wässrige Lösung, die den Polyester nach einem der Ansprüche 1 bis 8 enthält und die bevorzugt ein Kaschierklebstoff ist.

10. Eine Personal Care Formulierung, die den Polyester nach einem der Ansprüche 1 bis 8 enthält und die bevorzugt ein Haarfestiger ist.

11. Die Verwendung der wässrigen Lösung nach Anspruch 9 als Kaschierklebstoff.

12. Die Verwendung des Polyesters nach einem der Ansprüche 1 bis 8 oder der Formulierung nach Anspruch10 zur Festigung von Haaren.

13. Ein Verfahren zur Herstellung des Polyesters nach einem der Ansprüche 1 bis 8 umfassend
die Reaktion eines Gemisches enthaltend die Säurekomponente wie definiert unter A, die Di-ol/amin-komponente wie definiert unter B, optional die Hydroxycarbonsäuren wie definiert unter c1, optional die Aminocarbonsäuren und/oder deren cylischen Amide wie definiert unter c2 und den Verzweiger wie definiert unter E, wobei ein Zwischenprodukt erhalten wird,
das Entfernen, bevorzugt durch Destillation, eines ggf. vorhandenen Überschusses an Di-ol/amin-komponente wie definiert unter B von dem Zwischenprodukt, und
die Reaktion des Zwischenproduktes mit der Verbindung wie definiert unter D,
wobei a1 und a2 und a3 unabhängig voneinander jeweils als freie Dicarbonsäure oder als Dialkylester, insbesondere als Dimethylester, eingesetzt werden,
und wobei b1, b2, b3 und b4 nicht-derivatisiert eingesetzt werden wie unter b1, b2, b3 und b4 definiert,
und wobei c1 als freie Hydroxycarbonsäure eingesetzt wird oder in der Form gemäß Formel IIa oder IIb, 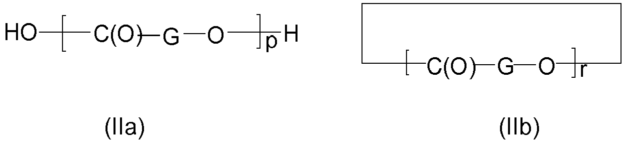 in denen p eine ganze Zahl von 1 bis 1500 und r eine ganze Zahl von 1 bis 4 bedeuten, und G für einen Rest steht, der ausgewählt ist aus der Gruppe bestehend aus Phenylen, -(CH₂)_{q}-, wobei q eine ganze Zahl von 1 bis 5 bedeutet, -C(R)H- und -C(R)HCH₂, wobei R für Methyl oder Ethyl steht,
und wobei c2 als freie Aminocarbonsäuren oder in der Form ihres cylischen Amids eingesetzt werden,
und wobei D nicht-derivatisiert als Di- oder Oligo-Isocyanat oder als Di- oder Oligo-Isocyanurat eingesetzt werden,
und wobei E nicht-derivatisiert eingesetzt wird.

## Claims

1. A polyester consisting of:
A) repeat units derived from an acid component, which consists of
a1) 30 to 90 mol%, based on the sum of components a1, a2, and a3, of 2,5-furandicarboxylic acid,
a2) 0 to 60 mol%, preferably 0 to 50 mol%, based on the sum of components a1, a2, and a3, of an aliphatic C₄-C₃₆ dicarboxylic acid or a mixture of a plurality of aliphatic C₄-C₃₆ dicarboxylic acids, and
a3) 10 to 60 mol%, based on the sum of components a1, a2, and a3, of a sulfonate group-containing dicarboxylic acid,
wherein the molar percentages of components a1) to a3) together add up to 100 mol%,
wherein preferably the proportion of a1 is 55 to 85 mol% and the proportion of a2 is at the same time 0 to 30 mol% and the proportion of a3 is at the same time 15 to 45 mol%,
B) repeat units derived from a di-ol/amine component consisting of
b1) a C₂ to C₁₂ alkanediol or a mixture of a plurality of C₂ to C₁₂ alkanediols, and/or
b2) a dihydroxy compound of formula I,
**HO-[(CH₂)ₙ-O]ₘ₋H** **(I)**
where n is 2, 3 or 4 and m is an integer from 2 to 250,
or a mixture of a plurality of such dihydroxy compounds,
b3) optionally one or more amino C₂ to C₁₂ alkanols or one or more amino C₅ to C₁₀ cycloalkanols or mixtures thereof, and
b4) optionally one or more diamino C₁ to C₈ alkanes,
wherein the amount of B is chosen so that for every 100 mol of A there is 100 mol of B present, minus the number of moles of D and E,
and wherein the sum of the proportions of b1 and of b2 in B is at least 50 mol%, preferably at least 80 mol%,
C) optionally repeat units derived from
c1) one or more hydroxycarboxylic acids of formula IIa,
HO-C(O)-G-OH (IIa)
where G represents a radical selected from the group consisting of phenylene, -(CH₂)_{q}-, in which q is an integer from 1 to 5, - C(R)H-, and -C(R)HCH₂, in which R is methyl or ethyl, and/or
c2) one or more aminocarboxylic acids or cyclic amides thereof selected from the group consisting of caprolactam, 1,6-aminocaproic acid, laurolactam, 1,12-aminolauric acid, and 1,11-aminoundecanoic acid,
wherein the proportion of C in the total mass of the polyester is preferably not more than 20% by weight, especially preferably not more than 10% by weight,
D) 0.1 to 4% by weight, based on the sum of components A, B, and C,
of repeat units derived from at least one di- or oligofunctional compound selected from the group consisting of a di- or oligoisocyanate and a di- or oligoisocyanurate, and
E) 0 to 4% by weight, based on the sum of components A, B, and C, of repeat units derived from a branching component that contains at least three functional groups, wherein the functional groups are selected from the group consisting of COOH, OH, NH₂, and mixtures thereof.

2. The polyester according to claim 1, wherein the sum of the proportions of a2, b3, b4, c1, and c2, based on the total mass of the polyester, is not more than 10% by weight, preferably not more than 5% by weight, and especially preferably 0% by weight.

3. The polyester according to claim 1 or 2, wherein a3 is an aromatic sulfonate group-containing dicarboxylic acid in which the sulfonate group is present either as the free acid or as a salt, preferably as an alkali metal salt and in particular as the sodium salt.

4. The polyester according to claim 3, wherein a3 is selected from the group consisting of 1,4-benzenedicarboxylic acid-2-sulfonic acid, 1,3-benzenedicarboxylic acid-5-sulfonic acid (also known as isophthalic acid-5-sulfonic acid), 1,2-benzenedicarboxylic acid-3-sulfonic acid, and 1,2-benzenedicarboxylic acid-4-sulfonic acid, in each case in the form of the free acid or of a salt, preferably in the form of an alkali metal salt, in particular in the form of the sodium salt, with the monosodium salt of isophthalic acid-5-sulfonic acid particularly preferred.

5. The polyester according to any of claims 1 to 4, wherein b1 is selected from the group consisting of ethylene glycol, 1,2-propanediol, 1,3-propanediol, 1,2-butanediol, 1,4-butanediol, 1,5-pentanediol, 2,4-dimethyl-2-ethylhexane-1,3-diol, 2,2-dimethyl-1,3-propanediol, 2-ethyl-2-butyl-l,3-propanediol, 2-ethyl-2-isobutyl-l,3-propanediol, 1,6-hexanediol, 1,8-octanediol, and 2,2,4-trimethyl-1,6-hexanediol, with b1 preferably selected from the group consisting of ethylene glycol, 1,4-butanediol, 1,6-hexanediol, and 1,8-octanediol.

6. The polyester according to any of claims 1 to 5, wherein b2 is selected from the group consisting of diethylene glycol, triethylene glycol, tetraethylene glycol, polyethylene glycol, polypropylene glycol, and polytetrahydrofuran, with b2 preferably selected from the group consisting of diethylene glycol, triethylene glycol, and tetraethylene glycol.

7. The polyester according to any of claims 1 to 6, wherein D is repeat units derived from an aliphatic diisocyanate, wherein the diisocyanate is preferably a straight-chain or branched alkylene diisocyanate or cycloalkylene diisocyanate having in both cases 2 to 20, in particular 3 to 15, carbon atoms, and wherein D is especially preferably 1,6-hexamethylene diisocyanate.

8. The polyester according to any of claims 1 to 7, wherein E is repeat units derived from a branching component selected from the group consisting of tartaric acid, citric acid, malic acid, trimethylolpropane, trimethylolethane, pentaerythritol, a polyether triol, glycerol, trimesic acid, trimellitic acid, and pyromellitic acid, wherein E is preferably repeat units derived from trimethylolpropane, pentaerythritol or glycerol, and wherein E is especially preferably repeat units derived from glycerol.

9. An aqueous solution comprising the polyester according to any of claims 1 to 8, and which is preferably a laminating adhesive.

10. A personal care formulation comprising the polyester according to any of claims 1 to 8, and which is preferably a hair-setting composition.

11. The use of the aqueous solution according to claim 9 as a laminating adhesive.

12. The use of the polyester according to any of claims 1 to 8, or of the formulation according to claim 10, for setting hair.

13. A process for production of the polyester according to any of claims 1 to 8 comprising
the reaction of a mixture comprising the acid component as defined under A, the di-ol/amine component as defined under B, optionally the hydroxycarboxylic acids as defined under c1, optionally the aminocarboxylic acids and/or cyclic amides thereof as defined under c2, and the branching component as defined under E, wherein an intermediate is obtained,
the removal from the intermediate, preferably through distillation, of any excess of the di-ol/amine component as defined under B, and
the reaction of the intermediate with the compound as defined under D,
wherein a1, a2, and a3 are independently each used as the free dicarboxylic acid or as the dialkyl ester, particularly as the dimethyl ester,
and wherein b1, b2, b3, and b4 are used underivatized as defined under b1, b2, b3, and b4,
and wherein c1 is used as the free hydroxycarboxylic acid or in the form shown in formula IIa or IIb, 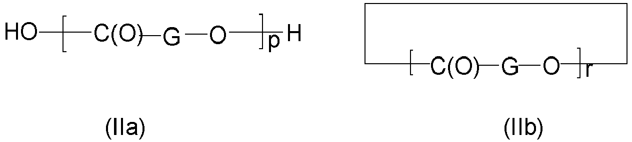 in which p is an integer from 1 to 1500 and r is an integer from 1 to 4, and G represents a radical selected from the group consisting of phenylene, -(CH₂)_{q}-, in which q is an integer from 1 to 5, -C(R)H-, and -C(R)HCH₂, in which R is methyl or ethyl,
and wherein c2 is used as the free aminocarboxylic acids or in the form of the cyclic amide thereof,
and wherein D is used underivatized as the di- or oligoisocyanate or as the di- or oligoisocyanurate,
and wherein E is used underivatized.

## Revendications

1. Polyester constitué :
A) de motifs répétitifs issus d'un composant de type acide, qui est constitué
a1) de 30 à 90 % en moles, par rapport à la somme des composants a1, a2 et a3, d'acide 2,5-furannedicarboxylique,
a2) de 0 à 60 % en moles, préférablement 0 à 50 % en moles, par rapport à la somme des composants a1, a2 et a3, d'un acide dicarboxylique aliphatique en C₄₋₃₆ ou d'un mélange de plusieurs acides dicarboxyliques aliphatiques en C₄₋₃₆, et
a3) de 10 à 60 % en moles, par rapport à la somme des composants a1, a2 et a3, d'un acide dicarboxylique contenant un groupe sulfonate,
les pourcentages en moles des composants a1) à a3) donnant ensemble 100 % en moles,
préférablement la proportion de a1 étant de 55 à 85 % en moles et en même temps la proportion de a2 étant de 0 à 30 % en moles et en même temps la proportion de a3 étant de 15 à 45 % en moles,
B) de motifs répétitifs issus d'un composant de type di-ol/amine constitué
b1) d'un C₂₋₁₂-alcanediol ou d'un mélange de plusieurs C₂₋₁₂-alcanediols, et/ou
b2) d'un composé de type dihydroxy de formule I,
**HO-[(CH₂)ₙ-O]ₘ-H** **(I)**
dans laquelle n représente 2, 3 ou 4 et m représente un nombre entier de 2 à 250,
ou d'un mélange de plusieurs de tels composés de type dihydroxy,
b3) éventuellement d'un ou plusieurs amino-C₂₋₁₂-alcanols ou d'un ou plusieurs amino-C₅₋₁₀-cycloalcanols ou de mélanges correspondants, et
b4) éventuellement d'un ou plusieurs diamino-C₁₋₈-alcanes,
la quantité de B étant choisi de telle manière que par 100 moles de A, 100 mol de B sont présentes, moins la quantité de moles de D et E, et la somme des proportions de b1 et de b2 par rapport à B étant d'au moins 50 % en moles, préférablement d'au moins 80 % en moles,
C) éventuellement de motifs répétitifs issus
c1) d'un ou plusieurs acides hydroxycarboxyliques de formule IIa,
**HO-C(O)-G-OH** **(IIa)**
dans laquelle G représente un radical qui est choisi dans le groupe constitué par phénylène, -(CH₂)_{q}-, q signifiant un nombre entier de 1 à 5, -C(R)H- et -C(R)HCH₂, R représentant méthyle ou éthyle, et/ou
c2) d'un ou plusieurs acides aminocarboxyliques ou de leurs amides cycliques choisis dans le groupe constitué par le caprolactame, l'acide 1,6-aminocaproïque, le laurolactame, l'acide 1,12-aminolaurique et l'acide 1,11-aminoundécanoïque,
préférablement la proportion de C dans la masse totale du polyester étant au maximum de 20 % en poids, en particulier préférablement au maximum de 10 % en poids,
D) 0,1 à 4 % en poids, par rapport à la somme des composants A et B et C,
de motifs répétitifs issus d'au moins un composé difonctionnel ou oligofonctionnel choisi dans le groupe constitué par un diisocyanate et un oligoisocyanate et un diisocyanurate et un oligoisocyanurate, et
E) 0 à 4 % en poids, par rapport à la somme des composants A et B et C,
de motifs répétitifs issus d'un agent de ramification qui contient au moins trois groupes fonctionnels, les groupes fonctionnels étant choisis dans le groupe constitué par COOH, OH, NH₂ et des mélanges correspondants.

2. Polyester selon la revendication 1, la somme des proportions de a2, b3, b4, c1 et c2, par rapport à la masse totale du polyester, étant au maximum de 10 % en poids, préférablement au maximum de 5 % en poids et en particulier préférablement 0 % en poids.

3. Polyester selon la revendication 1 ou 2, a3 étant un acide dicarboxylique contenant un groupe sulfonate aromatique dans lequel le groupe sulfonate est présent en tant qu'acide libre ou en tant que sel, préférablement en tant que sel de métal alcalin et en particulier en tant que sel de sodium.

4. Polyester selon la revendication 3, a3 étant choisi dans le groupe constitué par l'acide 1,4-benzènedicarboxylique-2-sulfonique, l'acide 1,3-benzènedicarboxylique-5-sulfonique (également appelé acide isophtalique-5-sulfonique), l'acide 1,2-benzènedicarboxylique-3-sulfonique, et l'acide 1,2-benzènedicarboxylique-4-sulfonique, à chaque fois sous forme d'acide libre ou d'un sel, préférablement sous forme d'un sel de métal alcalin, en particulier sous forme d'un sel de sodium, l'acide isophtalique-5-sulfonique monosodique étant particulièrement préféré.

5. Polyester selon l'une quelconque des revendications 1 à 4, b1 étant choisi dans le groupe constitué par l'éthylèneglycol, le 1,2-propanediol, le 1,3-propanediol, le 1,2-butanediol, le 1,4-butanediol, le 1,5-pentanediol, le 2,4-diméthyl-3-éthylhexane-l,3-diol, le 2,2-diméthyl-1,3-propanediol, le 2-éthyl-2-butyl-1,3-propanediol, le 2-éthyl-2-isobutyl-1,3-propanediol, le 1,6-hexanediol, le 1,8-octanediol et le 2,2,4-triméthyl-1,6-hexanediol, b1 étant préférablement choisi dans le groupe constitué par l'éthylèneglycol, le 1,4-butanediol, le 1,6-hexanediol et le 1,8-octanediol.

6. Polyester selon l'une quelconque des revendications 1 à 5, b2 étant choisi dans le groupe constitué par le diéthylèneglycol, le triéthylèneglycol, le tétraéthylèneglycol, un polyéthylèneglycol, un polypropylèneglycol et un polytétrahydrofuranne, b2 étant préférablement choisi dans le groupe constitué par le diéthylèneglycol, le triéthylèneglycol et le tétraéthylèneglycol.

7. Polyester selon l'une quelconque des revendications 1 à 6, des motifs répétitifs D étant issus d'un diisocyanate aliphatique, le diisocyanate étant préférablement un alkylènediisocyanate linéaire ou ramifié ou un cycloalkylènediisocyanate, comportant dans les deux cas 2 à 20, en particulier 3 à 15, atomes de carbone, et D étant en particulier préférablement le 1,6-diisocyanate d'hexamethylène.

8. Polyester selon l'une quelconque des revendications 1 à 7, des motifs répétitifs E étant issus d'un agent de ramification choisi dans le groupe constitué par l'acide tartrique, l'acide citrique, l'acide malique, le triméthylolpropane, le triméthyloléthane, le pentaérythritol, un polyéthertriol, la glycérine, l'acide trimésique, l'acide trimellitique et l'acide pyromellitique, préférablement des motifs répétitifs E étant issus du triméthylolpropane, du pentaérythritol ou de la glycérine, et en particulier préférablement des motifs répétitifs E étant issus de la glycérine.

9. Solution aqueuse qui contient le polyester selon l'une quelconque des revendications 1 à 8 et qui est préférablement un adhésif pour stratifié.

10. Formulation de soin personnel qui contient le polyester selon l'une quelconque des revendications 1 à 8 et qui est préférablement un fixateur capillaire.

11. Utilisation de la solution aqueuse selon la revendication 9 en tant qu'adhésif pour stratifié.

12. Utilisation du polyester selon l'une quelconque des revendications 1 à 8 ou de la formulation selon la revendication 10 pour la fixation de cheveux.

13. Procédé pour la préparation du polyester selon l'une quelconque des revendications 1 à 8 comprenant
la réaction d'un mélange contenant le composant de type acide tel que défini en A, le composant de type di-ol/amine tel que défini en B, éventuellement les acides hydroxycarboxyliques tels que définis en c1, éventuellement les acides aminocarboxyliques et/ou leurs amides cycliques tels que définis en c2 et l'agent de ramification tel que défini en E, un produit intermédiaire étant obtenu,
l'élimination, préférablement par distillation, d'un excès éventuellement présent du composant de type di-ol/amine tel que défini en B, du produit intermédiaire, et
la réaction du produit intermédiaire avec le composé tel que défini en D,
a1 et a2 et a3 étant utilisés indépendamment les uns des autres à chaque fois en tant qu'acide dicarboxylique libre ou en tant qu'ester de dialkyle, en particulier en tant qu'ester de diméthyle,
et b1, b2, b3 et b4 étant utilisés sous forme non dérivatisée, tel que défini en b1, b2, b3 et b4,
et c1 étant utilisé en tant qu'acide hydroxycarboxylique libre ou sous une forme selon la formule IIa ou IIb, 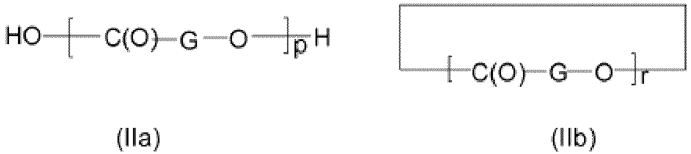 dans lesquelles p signifie un nombre entier de 1 à 1 500 et r signifie un nombre entier de 1 à 4, et G représente un radical qui est choisi dans le groupe constitué par phénylène, - (CH₂)_{q}-, q signifiant un nombre entier de 1 à 5, -C(R)H- et -C(R)HCH₂, R représentant méthyle ou éthyle,
et c2 étant utilisé en tant qu'acides aminocarboxyliques libres ou sous forme de leurs amides cycliques,
et D étant utilisé, sous forme non dérivatisée, en tant que diisocyanate ou oligoisocyanate ou en tant que diisocyanurate ou oligoisocyanurate,
et E étant utilisé sous forme non dérivatisée.
